Europäisches Patentamt
European Patent Office
Office européen des brevets

(11) Veröffentlichungsnummer: **0 233 341 A1**

(12) **EUROPÄISCHE PATENTANMELDUNG**

(21) Anmeldenummer: 86117017.3

(22) Anmeldetag: 08.12.86

(51) Int.Cl.³: C 07 D 403/04
C 07 D 231/44, A 01 N 43/56
A 01 N 43/36, A 01 N 43/647

(30) Priorität: 19.12.85 DE 3545036

(43) Veröffentlichungstag der Anmeldung: 26.08.87 Patentblatt 87/35

(84) Benannte Vertragsstaaten: AT BE CH DE ES FR GB GR IT LI NL SE

(71) Anmelder: BAYER AG
Konzernverwaltung RP Patentabteilung
D-5090 Leverkusen 1 Bayerwerk(DE)

(72) Erfinder: Jensen-Korte, Uta, Dr.
Geibelstrasse 9
D-4000 Duesseldorf 1(DE)

(72) Erfinder: Gehring, Reinhold, Dr.
Dasnoeckel 49
D-5600 Wuppertal 11(DE)

(72) Erfinder: Schallner, Otto, Dr.
Noldeweg 22
D-4019 Monheim(DE)

(72) Erfinder: Stetter, Jörg, Dr.
Gellertweg 4
D-5600 Wuppertal 1(DE)

(72) Erfinder: Wroblowsky, Heinz-Jürgen, Dr.
Gladbacher Strasse 34
D-4018 Langenfeld(DE)

(72) Erfinder: Becker, Benedikt, Dr.
Metzkausenerstrasse 14
D-4020 Mettmann(DE)

(72) Erfinder: Homeyer, Bernhard, Dr.
Obere Strasse 28
D-5090 Leverkusen(DE)

(72) Erfinder: Behrenz, Wolfgang, Dr.
Untergruendemich 14
D-5063 Overath(DE)

(72) Erfinder: Stendel, Wilhelm, Dr.
In den Birken 55
D-5600 Wuppertal 1(DE)

(54) **5-Heterocyclyl-1-aryl-pyrazole.**

(57) Es werden neue 5-Heterocyclyl-1-aryl-pyrazole der allgemeinen Formel (I),

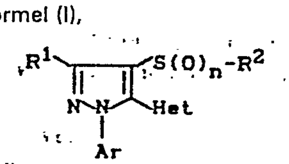

(I)

bereitgestellt,

in welcher

$R^1$ für Wasserstoff, Alkyl oder Halogenalkyl steht,

$R^2$ für Alkyl, Alkenyl, Alkinyl, Cycloalkyl, Halogenalkyl, Halogenalkenyl, für gegebenenfalls substituiertes Aralkyl oder für gegebenenfalls substituiertes Aryl steht,

Ar für jeweils gegebenenfalls substituiertes Phenyl oder Pyridyl steht,

Het für einen Heterocyclus der Formel

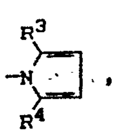
,
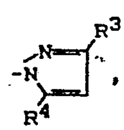
, oder
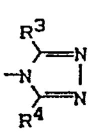
steht und

n für eine Zahl 0, 1 oder 2 steht,

wobei

$R^3$ und $R^4$ unabhängig voneinander jeweils für Wasserstoff, Alkyl oder Phenyl stehen.

Die neuen Verbindungen der Formel (I) besitzen eine stark ausgeprägte Wirksamkeit gegen tierische Schädlinge und insbesondere gegen Insekten.

EP 0 233 341 A1

0233341

BAYER AKTIENGESELLSCHAFT 5090 Leverkusen, Bayerwerk
Konzernverwaltung RP
Patentabteilung Er/li-c
Typ Ib

5-Heterocyclyl-1-aryl-pyrazole

Die Erfindung betrifft neue 5-Heterocyclyl-1-aryl-pyrazole, mehrere Verfahren zu ihrer Herstellung sowie ihre Verwendung als Schädlingsbekämpfungsmittel.

Es ist bereits bekannt, daß Pyrazolderivate, wie beispielsweise das 1-Cyclohexyl-5-[N,N-(dimethyl)-Carbamoyloxy]-3-methylthiomethyl-pyrazol oder das 1-Cyclohexyl-5-[N,N-(dimethyl)-Carbamoyloxy]-3-methylsulfonylmethyl-pyrazol insektizide Eigenschaften besitzen (vgl. z.B. DE-OS 2 839 270.

Die Wirkungshöhe bzw. die Wirkungsdauer dieser Verbindungen sind jedoch, insbesondere bei bestimmten Insekten oder bei niedrigen Anwendungskonzentrationen nicht immer völlig zufriedenstellend.

Es wurden neue 5-Heterocyclyl-1-aryl-pyrazole der allgemeinen Formel (I),

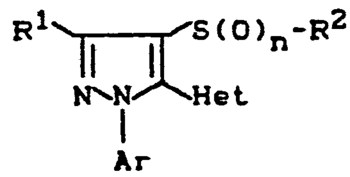 (I)

in welcher

$R^1$ für Wasserstoff, Alkyl oder Halogenalkyl steht,

$R^2$ für Alkyl, Alkenyl, Alkinyl, Cycloalkyl, Halogenalkyl, Halogenalkenyl, für gegebenenfalls substituiertes Aralkyl oder für gegebenenfalls substituiertes Aryl steht,

Ar für jeweils gegebenenfalls substituiertes Phenyl oder Pyridyl steht,

Het für einen Heterocyclus der Formel

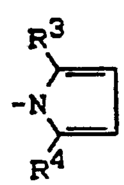 , 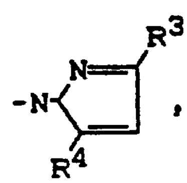 , oder 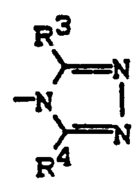 steht und

n für eine Zahl 0, 1 oder 2 steht,

wobei

$R^3$ und $R^4$ unabhängig voneinander jeweils für Wasserstoff, Alkyl oder Phenyl stehen,

gefunden.

Weiterhin wurde gefunden, daß man die neuen 5-Heterocyclyl-1-aryl-pyrazole der allgemeinen Formel (I)

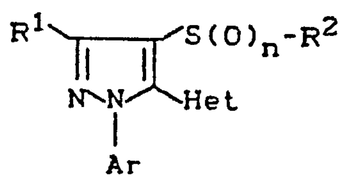

(I)

in welcher

$R^1$ für Wasserstoff, Alkyl oder Halogenalkyl steht,

$R^2$ für Alkyl, Alkenyl, Alkinyl, Cycloalkyl, Halogenalkyl, Halogenalkenyl, für gegebenenfalls substituiertes Aralkyl oder für gegebenenfalls substituiertes Aryl steht,

Ar für jeweils gegebenenfalls substituiertes Phenyl oder Pyridyl steht,

Het für einen Heterocyclus der Formel

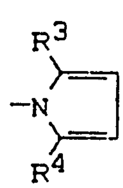 , 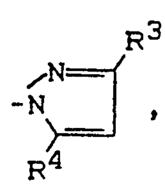 , oder 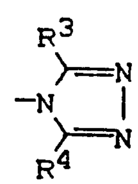 steht und

n für eine Zahl 0, 1 oder 2 steht,

wobei

$R^3$ und $R^4$ unabhängig voneinander jeweils für Wasserstoff, Alkyl oder Phenyl stehen,

mit Hilfe eines der im folgenden beschriebenen Herstellungsverfahren erhält:

a) Man erhält 5-Pyrrolyl-1-aryl-pyrazole der Formel (Ia),

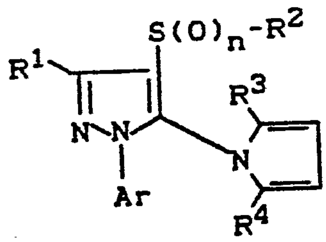

(Ia)

in welcher

$R^1$, $R^2$, $R^3$, $R^4$, n und Ar die oben angegebene Bedeutung haben,

wenn man 5-Amino-1-aryl-pyrazole der Formel (II)

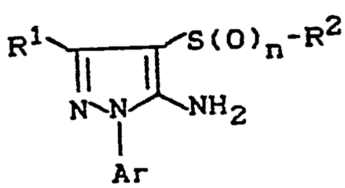

(II)

in welcher

$R^1$, $R^2$, Ar und n die oben angegebene Bedeutung haben,

α) mit 1,4-Dicarbonylverbindungen der Formel (IIIa),

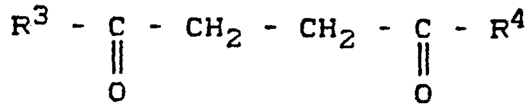 (IIIa)

in welcher

$R^3$ und $R^4$ die oben angegebene Bedeutng haben,

oder

β) mit deren Acetalen oder Ketalen der Formel (IIIb),

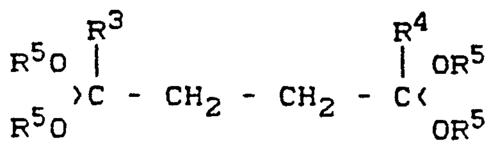 (IIIb)

in welcher

$R^3$ und $R^4$ die oben angegebene Bedeutung haben und

$R^5$ für Alkyl steht,

oder

γ) mit deren cyclischen Acetalstrukturen der Formel (IIIc),

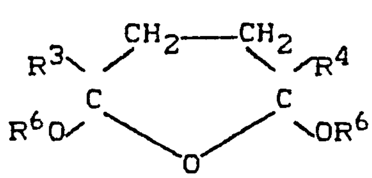 (IIIc)

in welcher

$R^3$ und $R^4$ die oben angegebene Bedeutung haben und

$R^6$ für Alkyl steht,

jeweils gegebenenfalls in Gegenwart eines Verdünnungsmittels und gegebenenfalls in Gegenwart eines Reaktionshilfsmittels umsetzt;

b) man erhält 5-(Pyrazol-1-yl)-1-aryl-pyrazole der Formel (Ib),

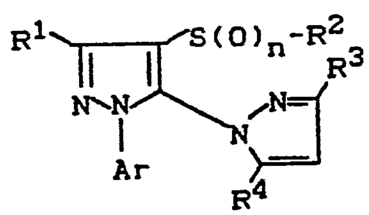

(Ib)

in welcher

$R^1$, $R^2$, $R^3$, $R^4$, Ar und n die oben angegebene Bedeutung haben,

wenn man 5-Hydrazino-1-aryl-pyrazole der Formel (IV),

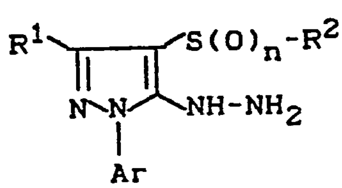

(IV)

in welcher

$R^1$, $R^2$, Ar und n die oben angegebene Bedeutung haben,

α) mit 1,3-Dicarbonylverbindungen der Formel (Va),

$$R^3 - \underset{\underset{O}{\|}}{C} - CH_2 - \underset{\underset{O}{\|}}{C} - R^4 \qquad (Va)$$

in welcher

$R^3$ und $R^4$ die oben angegebene Bedeutung haben,

oder

β) mit deren Acetalen oder Ketalen der Formel (Vb),

$$\begin{matrix} R^5O & & R^3 & & & & R^4 & & OR^5 \\ & \rangle & \overset{|}{C} & - & CH_2 & - & \overset{|}{C} & \langle & \\ R^5O & & & & & & & & OR^5 \end{matrix} \qquad (Vb)$$

in welcher

$R^3$, $R^4$ und $R^5$ die oben angegebene Bedeutung haben,

jeweils gegebenenfalls in Gegenwart eines Verdünnungsmittels und gegebenenfalls in Gegenwart eines Reaktionshilfsmittels umsetzt;

c) man erhält 5-(1,3,4-Triazol-1-yl)-1-aryl-pyrazole der Formel (Ic),

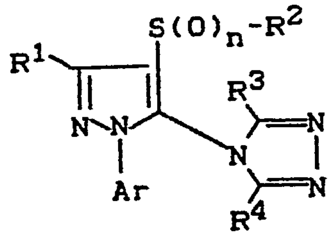

(Ic)

in welcher R', $R^2$, $R^3$, $R^4$, Ar und n die oben angegebene Bedeutung haben,

wenn man 5-Amino-1-aryl-pyrazole der Formel (II),

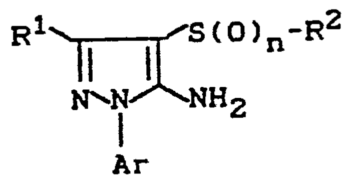

(II)

in welcher

$R^1$, $R^2$, Ar und n die oben angegebene Bedeutung haben,

mit Diacyl-Hydrazinen der Formel (VI),

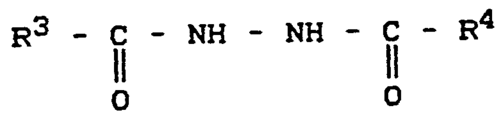

(VI))

in welcher

$R^3$ und $R^4$ die oben angegebene Bedeutung haben,

gegebenenfalls in Gegenwart eines Verdünnungsmittels umsetzt.

Schließlich wurde gefunden, daß die neuen 5-Heterocyclyl-1-aryl-pyrazole der allgemeinen Formel (I) sehr stark ausgeprägte pestizide und insbesondere insektizide Eigenschaften besitzen.

Überraschenderweise zeigen die erfindungsgemäßen 5-Heterocyclyl-1-aryl-pyrazole der allgemeinen Formel (I) eine erheblich bessere insektizide Wirksamkeit als die aus dem Stand der Technik bekannten Pyrazolderivate, wie beispielsweise das 1-Cyclohexyl-5-[N,N-(dimethyl)-carbamoyloxy]-3-methylthiomethyl-pyrazol oder das 1-Cyclohexyl-5-[N,N-(dimethyl)-carbamoyloxy]-3-methylsulfonyl-pyrazol, welches chemisch und wirkungsmäßig naheliegende Verbindungen sind.

Die erfindungsgemäßen 5-Heterocyclyl-1-aryl-pyrazole sind durch die Formel (I) allgemein definiert.

Bevorzugt sind diejenigen Verbindungen der Formel (I), bei welchen

$R^1$ für jeweils geradkettiges oder verzweigtes Alkyl oder Halogenalkyl mit 1 bis 4 Kohlenstoffatomen und gegebenenfalls 1 bis 9 gleichen oder verschiedenen Halogenatomen oder für Wasserstoff steht,

$R^2$ für jeweils geradkettiges oder verzweigtes Alkyl, Alkenyl oder Alkinyl mit jeweils bis zu 8 Kohlenstoffatomen, für jeweils geradkettiges oder verzweigtes Halogenalkyl oder Halogenalkenyl mit jeweils bis zu 8 Kohlenstoffatomen und bis zu 17 gleichen oder

verschiedenen Halogenatomen, für Cycloalkyl mit 3 bis 7 Kohlenstoffatomen oder für jeweils gegebenenfalls einfach oder mehrfach, gleich oder verschieden substituiertes Phenylalkyl oder Phenyl mit gegebenenfalls 1 bis 4 Kohlenstoffatomen im geradkettigen oder verzweigten Alkylteil steht, wobei als Phenylsubstituenten jeweils in Frage kommen: Halogen, Cyano, Nitro, jeweils geradkettiges oder verzweigtes Alkyl, Alkoxy, Alkylthio, Alkylsulfinyl, Alkylsulfonyl, Halogenalkyl, Halogenalkoxy, Halogenalkylthio, Halogenalkylsulfinyl oder Halogenalkylsulfonyl mit jeweils 1 bis 4 Kohlenstoffatomen in den einzelnen Alkylteilen und gegebenenfalls 1 bis 9 gleichen oder verschiedenen Halogenatomen,

Ar für jeweils gegebenenfalls einfach oder mehrfach, gleich oder verschieden substituiertes Phenyl, 2-Pyridyl, 3-Pyridyl oder 4-Pyridyl steht, wobei als Substituenten in Frage kommen: Cyano, Nitro, Halogen, jeweils geradkettiges oder verzweigtes Alkyl, Alkoxy oder Alkoxycarbonyl mit jeweils bis zu 4 Kohlenstoffatomen, außerdem jeweils geradkettiges oder verzweigtes Halogenalkyl oder Halogenalkoxy mit jeweils bis zu 4 Kohlenstoffatomen und bis zu 9 gleichen oder verschiedenen Halogenatomen oder ein Rest $-S(O)_m-R^7$

wobei

$R^7$ für Amino, oder für jeweils geradkettiges oder verzweigtes Alkyl, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen steht und

m für eine Zahl 0, 1 oder 2 steht,

n ebenfalls für eine Zahl 0, 1 oder 2 steht und

Het für einen Rest 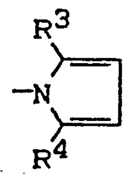 , 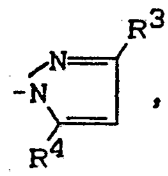 , oder 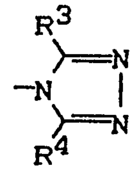

steht,

wobei

$R^3$ und $R^4$ unabhängig voneinander jeweils für Wasserstoff, für geradkettiges oder verzweigtes Alkyl mit 1 bis 4 Kohlenstoffatomen oder für Phenyl stehen.

Besonders bevorzugt sind diejenigen Verbindungen der Formel (I), bei welchen

$R^1$ für Wasserstoff, Methyl, Ethyl, n- oder i-Propyl oder Trifluormethyl steht,

$R^2$ für Methyl, Ethyl, n- oder i-Propyl, n-, i-, s- oder t-Butyl, n- oder i-Pentyl, n- oder i-Hexyl, Allyl, Propenyl, Butenyl, Propargyl, Butinyl, Cyclopropyl, Cyclopentyl, Cyclohexyl, Chlormethyl, Difluormethyl, Difluorchlormethyl, Fluordichlormethyl, Trifluormethyl, Pentafluorethyl, Pentachlorethyl, Fluortetrachlorethyl, Difluortrichlorethyl, Trifluordichlorethyl, Tetrafluorchlorethyl, Heptafluorpropyl, Chlorethyl, Bromethyl, Chlorpropyl, Chlorpropenyl, Dichlorpropenyl, Chlorbutenyl oder für jeweils gegebenenfalls ein- bis dreifach, gleich oder verschieden substituiertes Phenyl, Benzyl oder Phenylethyl steht, wobei als Phenylsubstituenten in Frage kommen: Fluor, Chlor, Brom, Iod, Cyano, Nitro, Methyl, Ethyl, Methoxy, Methylthio, Trifluormethyl, Methylsulfinyl, Methylsulfonyl, Trifluormethoxy, Trifluormethylthio, Trifluormethylsulfinyl oder Trifluormethylsulfonyl,

Ar für jeweils gegebenenfalls ein- bis fünffach, gleich oder verschieden substituiertes Phenyl, 2-Pyridyl, 3-Pyridyl oder 4-Pyridyl steht, wobei als Substituenten in Frage kommen: Cyano, Nitro, Fluor, Chlor, Brom, Iod, Methyl, Ethyl, n- und i-Propyl, n-, i-, s- und t-Butyl, Methoxy, Ethoxy, Methoxycarbonyl, Ethoxycarbonyl, Trifluormethyl, Trichlormethyl, Difluorchlormethyl, Chlormethyl, Dichlormethyl, Difluormethyl, Pentafluorethyl, Tetrafluorethyl, Trifluorchlorethyl,Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_m-R^7$

wobei

$R^7$ für Amino, Methylamino, Ethylamino, Dimethylamino, Diethylamino, Fluordichlormethyl, Difluormethyl, Tetrafluorethyl, Trifluorchlorethyl, Trifluormethyl, Methyl oder Ethyl steht,

m für eine Zahl 0, 1 oder 2 steht,

n ebenfalls für eine Zahl 0, 1 oder 2 steht und

Het für einen Heterocyclus der Formel

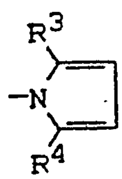 , 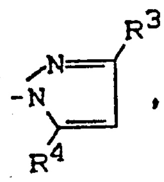 , oder 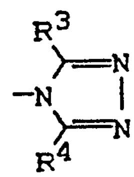 steht,

wobei

$R^3$ und $R^4$ unabhängig voneinander jeweils für Wasserstoff, Methyl, Ethyl, n- oder i-Propyl oder Phenyl stehen.

Im einzelnen seien außer den bei den Herstellungsbeispielen genannten Verbindungen die folgenden 5-Heterocyclyl-1-aryl-pyrazole der allgemeinen Formel (I) genannt:

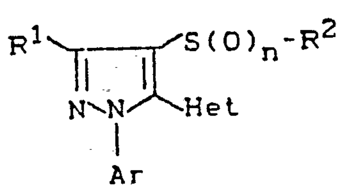

(I)

| $R^1$ | $-S(O)_n-R^2$ | Het | Ar |
|---|---|---|---|
| H | $SCF_3$ | 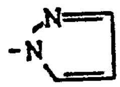 | 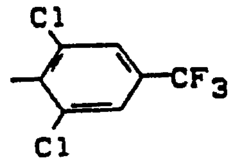 |
| H | $SCCl_2F$ | 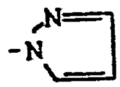 | 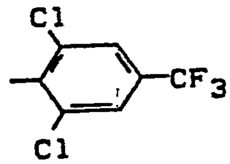 |
| H | $SCClF_2$ | 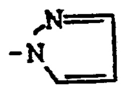 | 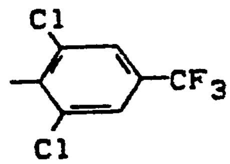 |
| H | $SCF_3$ | 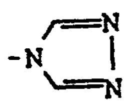 | 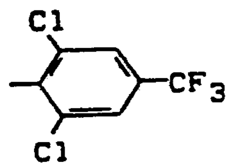 |
| H | $SCCl_2F$ | 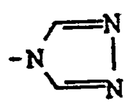 | 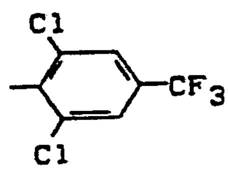 |
| H | $SCClF_2$ | 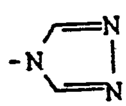 | 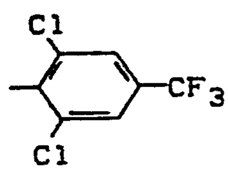 |
| H | $SCF_3$ | 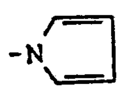 | 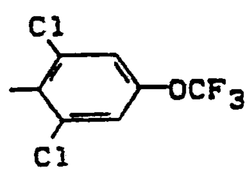 |

| $R^1$ | $-S(O)_n-R^2$ | Het | Ar |
|---|---|---|---|
| H | $SCCl_2F$ | 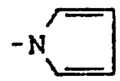 | 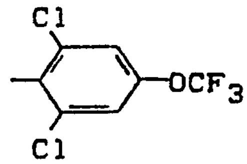 |
| H | $SCClF_2$ | 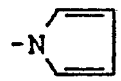 | 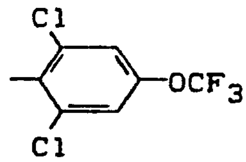 |
| H | $SCF_3$ | 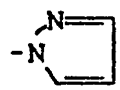 | 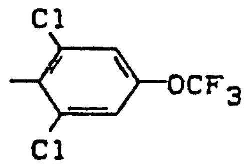 |
| H | $SCCl_2F$ | 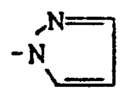 | 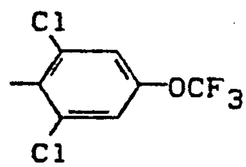 |
| H | $SCClF_2$ | 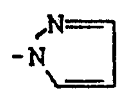 | 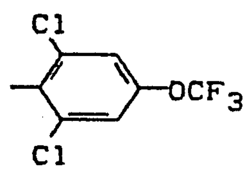 |
| H | $SCF_3$ | 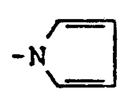 | 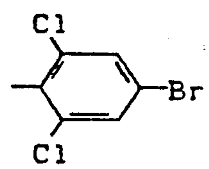 |

| $R^1$ | $-S(O)_nR^2$ | Het | Ar |
|---|---|---|---|
| H | $SCCl_2F$ | 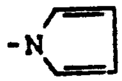 | 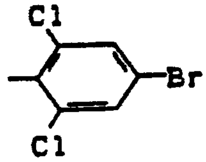 |
| H | $SCClF_2$ | 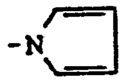 | 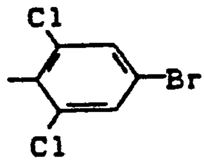 |
| H | $SO_2CF_3$ | 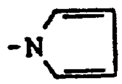 | 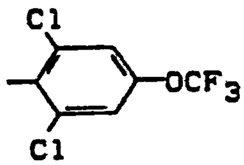 |
| H | $SO_2CCl_2F$ | 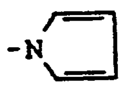 | 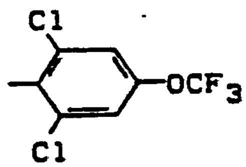 |
| H | $SO_2CClF_2$ | 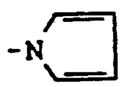 | 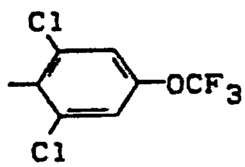 |
| H | $SO_2CF_3$ | 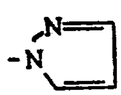 | 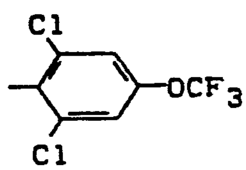 |

| $R^1$ | $-S(O)_nR^2$ | Het | Ar |
|---|---|---|---|
| H | $SO_2CCl_2F$ | 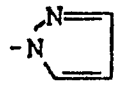 | 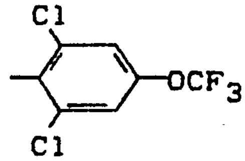 |
| H | $SO_2CClF_2$ | 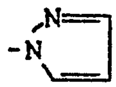 | 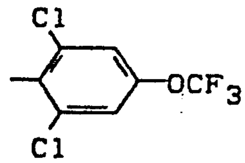 |
| $CH_3$ | $SCF_3$ | 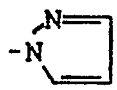 | 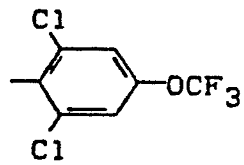 |
| $CH_3$ | $SCCl_2F$ | 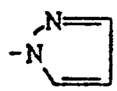 | 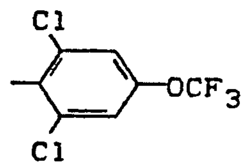 |
| $CH_3$ | $SCClF_2$ | 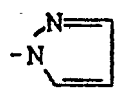 | 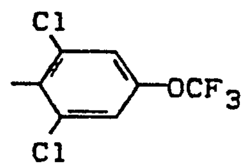 |
| $CH_3$ | $SCF_3$ | 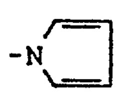 | 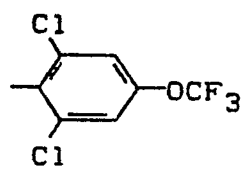 |

| $R^1$ | $-S(O)_nR^2$ | Het | Ar |
|---|---|---|---|
| $CH_3$ | $SCCl_2F$ | 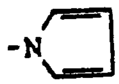 | 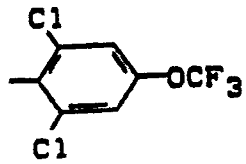 |
| $CH_3$ | $SCClF_2$ | 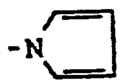 | 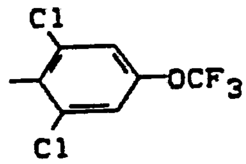 |
| $CH_3$ | $SO_2CF_3$ | 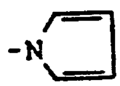 | 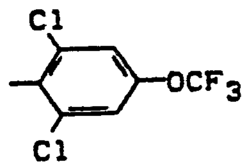 |
| $CH_3$ | $SO_2CCl_2F$ | 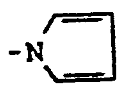 | 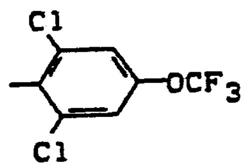 |
| $CH_3$ | $SO_2CClF_2$ | 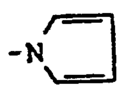 | 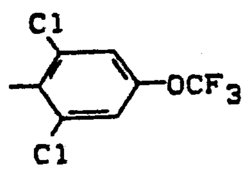 |

Verwendet man beispielsweise 5-Amino-1-phenyl-4-dichlorfluormethylsulfenyl-pyrazol und 2,5-Diethoxytetrahydrofuran als Ausgangsstoffe, so läßt sich der Reaktionsablauf des erfindungsgemäßen Verfahrens (a) durch das folgende Formelschema darstellen:

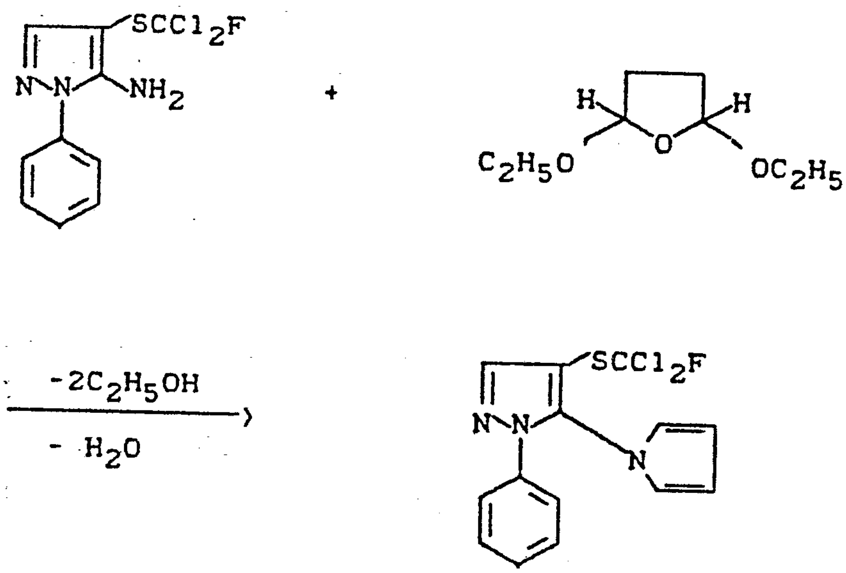


Verwendet man beispielsweise 1-(2,6-Dichlor-4-trifluormethyl-phenyl)-5-hydrazino-4-trifluormethylsufonyl-pyrazol und 1,1,3,3-Tetramethoxypropan als Ausgangsstoffe, so läßt sich der Reaktionsablauf des erfindungsgemäßen Verfahrens (b) durch das folgende Formelschema darstellen:

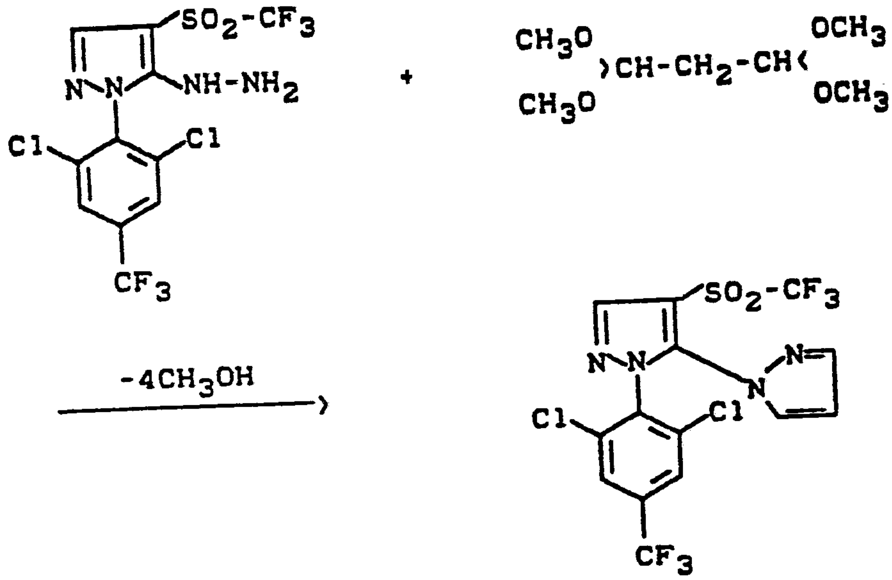


Verwendet man beispielsweise 5-Amino-4-methylthio-3-methyl-1-(2,4,6-trichlorphenyl)-pyrazol und 1,2-Dibenzoylhydrazin als Ausgangsstoffe, so läßt sich der Reaktionsablauf des erfindungsgemäßen Verfahrens (c) durch das folgende Formelschema darstellen:

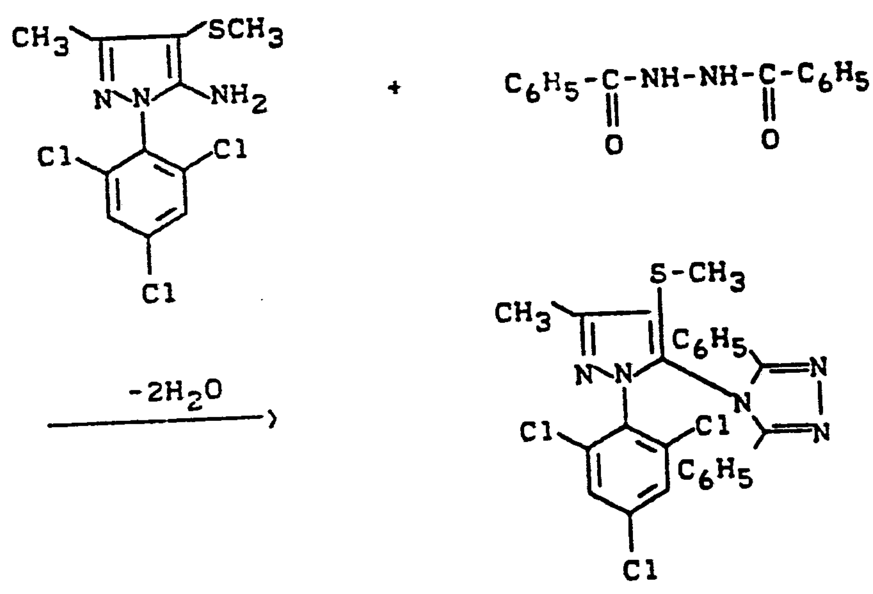

Die zur Durchführung der erfindungsgemäßen Verfahren (a) und (c) als Ausgangsstoffe benötigten 5-Amino-1-aryl-pyrazole sind durch die Formel (II) allgemein definiert. In dieser Formel (II) stehen

$R^1$, $R^2$, Ar und n vorzugsweise für diejenigen Reste, die bereits im Zusammenhang mit der Beschreibung der erfindungsgemäßen Stoffe der Formel (I) als bevorzugt für diese Substituenten genannt wurden.

Die 5-Amino-1-aryl-pyrazole der Formel (II) sind teilweise bekannt (vgl. z.B. Pharmaco. Ed. Sci. 26, 276-293 [1971] oder Mycopathologica 74, 7-14 [1981] vgl. auch C.A. 96: 196411j bzw. C.A. 95: 36257q; DE-OS 3 402 308); teilweise sind sie Gegenstand einer eigenen vorgängigen noch nicht publizierten Patentanmeldung (vgl. DE-P 3 517 843) und können in Analogie zu den dort beschriebenen Herstellungsverfahren erhalten werden,

beispielsweise, wenn man

4-Thiocyanato-5-aminopyrazole der allgemeinen Formel (VII),

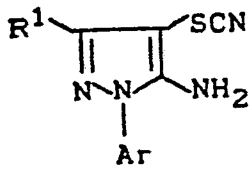

(VII)

in welcher

$R^1$ und Ar die oben angegebene Bedeutung haben,

oder

Bis-(pyrazolyl)-disulfide der Formel (VIII),

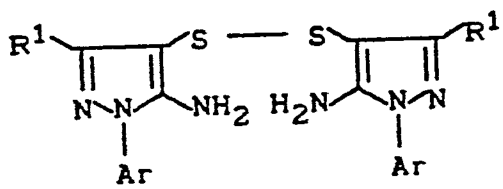

(VIII)

in welcher

$R^1$ und Ar die oben angegebene Bedeutung haben,

mit Halogeniden der Formel (IX),

$$R^2 - Hal \quad (IX)$$

in welcher

$R^2$ die oben angegebene Bedeutung hat und

Hal für Halogen insbesondere für Chlor oder Brom steht,

gegebenenfalls in Gegenwart eines Verdünnungsmittels wie beispielsweise Methanol sowie in Gegenwart eines geeigneten Reduktionsmittels wie beispielsweise Natriumborhydrid oder Natriumdithionit und in Gegenwart einer Base wie beispielsweise Kaliumhydroxid bei Temperaturen zwischen 20 °C und 90 °C umsetzt,

oder wenn man

4-unsubstituierte 5-Amino-pyrazole der Formel (X),

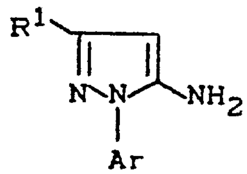

(X)

in welcher

$R^1$ und Ar die oben angegebenen Bedeutungen haben,

mit Sulfenylhalogeniden der Formel (XI),

$$R^2 - S - Hal' \qquad (XI)$$

in welcher

$R^2$ die oben angegebene Bedeutung hat und

Hal' für Halogen, insbesondere für Chlor oder Brom steht,

gegebenenfalls in Gegenwart eines Verdünnungsmittels wie beispielsweise Dichlormethan und gegebenenfalls in Gegenwart eines Säurebindemittels wie beispielsweise Pyridin bei Temperaturen zwischen 0 °C und 50 °C umsetzt;

oder wenn man

die nach den oben beschriebenen Verfahren erhältlichen 5-Aminopyrazole der Formel (IIa),

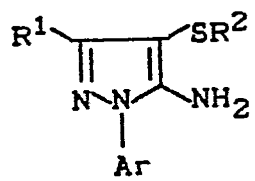

(IIa)

in welcher

$R^1$, $R^2$ und Ar die oben angegebene Bedeutung haben,

mit Oxidationsmitteln der Formel (XII),

$$R^8 - O - OH \qquad (XII)$$

in welcher

$R^8$ für Wasserstoff oder für jeweils gegebenenfalls substituiertes Alkanoyl oder Aroyl steht, insbesondere für Wasserstoff, für Acetyl, für Propionyl, für Trifluoracetyl oder für gegebenenfalls substituiertes Benzoyl, wie beispielsweise 3-Chlorbenzoyl oder 4-Nitrobenzoyl steht,

gegebenenfalls in Gegenwart eines Verdünnungsmittels wie beispielsweise Dichlormethan, gegebenenfalls in Gegenwart eines Katalysators wie beispielsweise Ammoniummolybdat und gegebenenfalls in Gegenwart eines Säurebindemittels wie beispielsweise Natriumcarbonat bei Temperaturen zwischen 0 °C und +50 °C am Schwefel der Sulfenylgruppe in 4-Position des Pyrazolringes oxidiert.

Die 4-Thiocyanato-5-aminopyrazole der Formel (VII) sind teilweise bekannt (vgl. z.B. Pharmaco Ed. Sci. 38, 274-282 [1983]). Man erhält sie beispielsweise, wenn man 4-unsubstituierte 5-Aminopyrazole der Formel (X),

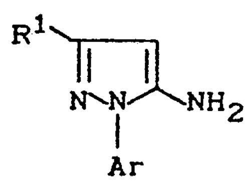

(X)

in welcher

$R^1$ und Ar die oben angegebene Bedeutung haben,

mit Ammoniumthiocyanat in Gegenwart von Brom und Essigsäure bei Temperaturen zwischen -20 °C und +20 °C umsetzt.

Die Bis-(pyrazolyl)-disulfide der Formel (VIII) sind noch nicht bekannt. Man erhält sie, wenn man die oben beschrieschriebenen 4-Thiocyanato-5-amino-pyrazole der Formel (VII) mit wässriger Salzsäure, gegebenenfalls in Gegenwart eines Verdünnungsmittels, wie beispielsweise Ethanol bei Temperaturen zwischen 20 °C und 120 °C umsetzt.

Die 4-unsubstituierten 5-Aminopyrazole der Formel (X) sind bekannt (vgl. z.B. J.Org.Chem. 36, 2972-2974 [1971] oder J. Heterocyclic Chemistry 7, 345-349 [1970]; C.A. 62: 13137c oder DE-OS 3 402 308) oder können nach bekannten Verfahren in einfacher analogern Weise erhalten werden (vgl. auch die Herstellungsbeispiele).

Die Halogenide der Formel (IX), die Sulfenylhalogenide der Formel (XI) und die Oxidationsmittel der Formel (XII) sind allgemein bekannte Verbindungen der organischen Chemie.

Die zur Durchführung des erfindungsgemäßen Verfahrens (a) weiterhin als Ausgangsstoffe benötigten 1,4-Dicarbonylverbindungen bzw. deren Acetale, Ketale oder cyclische Acetalstrukturen sind durch die Formeln (III a-c) allgemein definiert. In diesen Formeln (III a-c) stehen $R^3$ und $R^4$ vorzugsweise für diejenigen Reste, die bereits im Zusammenhang mit der Beschreibung der erfindungsgemäßen Stoffe der Formel (I) als bevorzugt für diese Substituenten genannt wurden.

In der Formel (IIIb) steht $R^5$ vorzugsweise für geradkettiges oder verzweigtes Alkyl mit 1 bis 4 Kohlenstoffatomen, insbesondere für Methyl oder Ethyl. In der Formel (IIIc) steht $R^6$ vorzugsweise ebenfalls für geradkettiges oder

verzweigtes Alkyl mit 1 bis 4 Kohlenstoffatomen, insbesondere für Methyl oder Ethyl.

Die 1,4-Dicarbonylverbindungen der Formel (IIIa), deren Acetale oder Ketale der Formel (IIIb) und deren cyclische Acetalstrukturen der Formel (IIIc) sind allgemein bekannte Verbindungen der organischen Chemie.

Die zur Durchführung des erfindungsgemäßen Verfahrens (b) als Ausgangsstoffe benötigten 5-Hydrazino-1-aryl-pyrazole sind durch die Formel (IV) allgemein definiert. In dieser Formel (IV) stehen $R^1$, $R^2$, Ar und n vorzugsweise für diejenigen Reste, die bereits im Zusammenhang mit der Beschreibung der erfindungsgemäßen Stoffe der Formel (I) als bevorzugt für diese Substituenten genannt wurden.

Die 5-Hydrazino-1-aryl-pyrazole der Formel (IV) sind noch nicht bekannt. Die Verbindungen der Formel (IV) zeigen ebenfalls eine stark ausgeprägte pestizide und insbesondere insektizide Wirksamkeit.

Man erhält sie, wenn man 5-Amino-1-aryl-pyrazole der Formel (II),

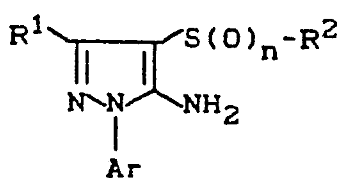

(II)

in welcher

$R^1$, $R^2$, Ar und n die oben angegebene Bedeutung haben,

zunächst in einer ersten Stufe mit Nitrit-Verbindungen der Formel (XIII),

$$R^9 - O - N = O \qquad (XIII)$$

in welcher

$R^9$ für Wasserstoff, Alkyl oder für ein Alkalimetallkation steht,

in Gegenwart einer Halogenwasserstoffsäure wie beispielsweise Chlorwasserstoffsäure oder Bromwasserstoffsäure oder in Gegenwart eines Haloforms wie beispielsweise Chloroform oder Bromoform bei Temperaturen zwischen -20 °C und +80 °C in üblicher Art und Weise diazotiert (vgl. z.B. "Organikum" 15. Auflage VEB Deutscher Verlag der Wissenschaften, Berlin, 1981, S. 652ff; J. Chem. Soc. C. 1966, 1249 oder Rev. Latinoam. Quim. 13, 100-102 [1982]) und die so erhältlichen 5-Halogen-1-aryl-pyrazole der Formel (XIV),

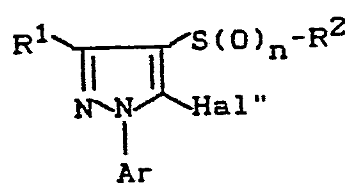

(XIV)

in welcher

$R^1$, $R^2$, Ar und n die oben angegebene Bedeutung haben und

Hal" für Halogen, insbesondere für Chlor oder Brom steht,

mit Hydrazinhydrat gegebenenfalls in Gegenwart eines Verdünnungsmittels, wie beispielsweise Dioxan bei Temperaturen zwischen 20 °C und 120 °C umsetzt.

Die Nitritverbindungen der Formel (XIII) sind allgemein bekannte Verbindungen der organischen Chemie.

Die zur Durchführung des erfindungsgemäßen Verfahrens (b) weiterhin als Ausgangsstoffe benötigten 1,3-Dicarbonylverbindungen bzw. deren Acetale oder Ketale sind durch die Formeln (Va) und (Vb) allgemein definiert. In diesen Formeln (Va) und (Vb) stehen $R^3$ und $R^4$ vorzugsweise für diejenigen Reste, die bereits im Zusammenhang mit der Beschreibung der erfindungsgemäßen Stoffe der Formel (I) als bevorzugt für diese Substituenten genannt wurden.

$R^5$ in der Formel (Vb) steht vorzugsweise für geradkettiges oder verzweigtes Alkyl mit 1 bis 4 Kohlenstoffatomen, insbesondere für Methyl oder Ethyl.

Die 1,3-Dicarbonylverbindungen der Formel (Va) und deren Acetale oder Ketale der Formel (Vb) sind allgemein bekannte Verbindungen der organischen Chemie.

Die zur Durchführung des erfindungsgemäßen Verfahrens (c) weiterhin als Ausgangsstoffe benötigten Diacylhydrazine sind durch die Formel (VI) allgemein definiert. In dieser

Formel (VI) stehen $R^3$ und $R^4$ vorzugsweise für diejenigen Reste, die bereits im Zusammenhang mit der Beschreibung der erfindungsgemäßen Stoffe der Formel (I) als bevorzugt für diese Substituenten genannt wurden.

Die Diacylhydrazine der Formel (VI) sind ebenfalls allgemein bekannte Verbindungen der organischen Chemie.

Als Verdünnungsmittel zur Durchführung des erfindungsgemäßen Verfahrens (a) kommen inerte organische Lösungsmittel infrage.

Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder diethylether, Alkohole wie Methanol, Ethanol oder Propanol, deren Gemische mit Wasser oder Säuren, wie Essigsäure oder deren Gemische mit Wasser.

Das erfindungsgemäße Verfahren (a) wird gegebenenfalls in Gegenwart eines geeigneten Reaktionshilfsmittels durchgeführt. Als solche verwendet man vorzugsweise anorganische oder organische Säuren, wie beispielsweise Essigsäure oder p-Toluolsulfonsäure.

Die Reaktionstemperaturen können bei der Durchführung des erfindungsgemäßen Verfahrens (a) in einem größeren Bereich

variiert werden. Im allgemeinen arbeitet man bei Temperaturen zwischen 20 °C und 200 °C, vorzugsweise bei Temperaturen zwischen 60 °C und 150 °C.

Zur Durchführung des erfindungsgemäßen Verfahrens (a) setzt man pro Mol an 5-Amino-1-aryl-pyrazol der Formel (II) im allgemeinen 1.0 bis 10.0 Mol, vorzugsweise 2.0 bis 5.0 Mol an 1,4-Dicarbonylderivat der Formel (IIIa), (IIIb) oder (IIIc) ein und gegebenenfalls 0.001 bis 10.0 Mol, vorzugsweise 0.01 bis 5.0 Mol an Reaktionshilfsmittel ein. Die Reaktionsdurchführung, Aufarbeitung und Isolierung der Reaktionsprodukte der Formel (Ia) erfolgt nach allgemein üblichen Methoden.

Als Verdünnungsmittel zur Durchführung des erfindungsgemäßen Verfahrens (b) kommen ebenfalls inerte organische Lösungsmittel infrage. Vorzugsweise verwendet man die bei Verfahren (a) aufgezählten Lösungsmittel.

Das erfindungsgemäße Verfahren (b) wird gegebenenfalls ebenfalls in Gegenwart eines geeigneten Reaktionshilfsmittels durchgeführt. Als solche verwendet man vorzugsweise die bei Verfahren (a) aufgeführten Reaktionshilfsmittel.

Die Reaktionstemperaturen können bei der Durchführung des erfindungsgemäßen Verfahrens (b) ebenfalls in einem größeren Bereich variiert werden. Im allgemeinen arbeitet man bei Temperaturen zwischen 0 °C und 200 °C, vorzugsweise bei Temperaturen zwischen 50 °C und 150 °C.

Zur Durchführung des erfindungsgemäßen Verfahrens (b)

setzt man pro Mol an 5-Hydrazino-1-aryl-pyrazol der Formel (IV) im allgemeinen 1.0 bis 10.0 Mol, vorzugsweise 2.0 bis 5.0 Mol an 1,3-Dicarbonylverbindung der Formel (Va) oder (Vb) und gegebenenfalls 0.001 bis 10.0 Mol, vorzugsweise 0.01 bis 5.0 Mol an Reaktionshilfsmittel ein.

Die Reaktionsdurchführung, Aufarbeitung und Isolierung der Reaktionsprodukte der Formel (Ib) erfolgt nach allgemein üblichen Methoden.

Als Verdünnungsmittel zur Durchführung des erfindungsgemäßen Verfahrens (c) kommen ebenfalls inerte organische Lösungsmittel infrage. Vorzugsweise verwendet man die bei Verfahren (a) aufgezählten Lösungsmittel.

Das erfindungsgemäße Verfahren (c) wird gegebenenfalls ebenfalls in Gegenwart eines geeigneten Reaktionshilfsmittels durchgeführt. Als solche verwendet man vorzugsweise die bei Verfahren (a) aufgeführten Reaktionshilfsmittel.

Die Reaktionstemperaturen können bei der Durchführung des erfindungsgemäßen Verfahrens (c) ebenfalls in einem größeren Bereich variiert werden. Im allgemeinen arbeitet man bei Temperaturen zwischen 0 °C und 200 °C, vorzugsweise bei Temperaturen zwischen 50 °C und 150 °C.

Zur Durchführung des erfindungsgemäßen Verfahrens (c) setzt man pro Mol an 5-Amino-1-aryl-pyrazol der Formel (II) im allgemeinen 1.0 bis 10.0 Mol, vorzugsweise

2,0 bis 5.0 Mol an Diacylhydrazin der Formel (VI) und gegebenenfalls 0.001 bis 10.0 Mol, vorzugsweise 0.01 bis 5 Mol an Reaktionshilfsmittel ein.

Die Reaktionsdurchführung, Aufarbeitung und Isolierung der Reaktionsprodukte der Formel (Ic) erfolgt nach allgemein üblichen Methoden.

Die Wirkstoffe gemäß den allgemeinen Formeln (I) und (IV) eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta

migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.

Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp.,

Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge, Flöhe.
Sie sind gegen normalsensible und resistente Arten und Stämme, sowie gegen alle parasitierenden und nicht parasitierenden Entwicklungsstadien der Ektoparasiten wirksam.

Die erfindungsgemäßen Wirkstoffe zeichnen sich durch eine hohe insektizide Wirksamkeit aus. Sie lassen sich mit besonders gutem Erfolg gegen pflanzenschädigende Insekten, wie beispielsweise gegen die Larven des Meerrettichblattkäfers (Phaedon cochleariae) sowie gegen die Raupen der Kohlschabe (Plutella maculipennis) einsetzen.

Daneben eignen sie sich auch hervorragend zur Bekämpfung von Bodeninsekten und lassen sich beispielsweise zur Bekämpfung von Phorbia antiqua Maden einsetzen.

Außerdem besitzen die erfindungsgemäßen Wirkstoffe eine hohe Wirkung gegen Hygiene- und Vorratsschädlinge und lassen sich beispielsweise zur Bekämpfung von Schaben (Blattella germanica), der Stubenfliege (Musca domestica) oder zur Bekämpfung des gemeinen Kornkäfers (Sitophilus granarius)einsetzen. Darüber hinaus lassen sich die erfindungsgemäßen Wirkstoffe mit besonders gutem Erfolg zur Bekämpfung von parasitisch lebenden Warmblüterschädlingen wie beispielsweise gegen die Larven der Goldfliege (Lucilia cuprina) einsetzen. Daneben besitzen die erfindungsgemäßen Wirkstoffe in entsprechenden Aufwandmengen auch eine gute fungizide Wirksamkeit und lassen sich beispielsweise zur Bekämpfung von Botrytis- und Venturia-Arten einsetzen. Auch die Zwischenprodukte der Formel (IV) zeigen gute insektizide Eigenschaften.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie

synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute

0233341

Alkalistabilität auf gekälkten Unterlagen aus.

Die erfindungsgemäß verwendbaren Wirkstoffe eignen sich auch zur Bekämpfung von Insekten, Milben, Zecken usw. auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer usw. erreicht werden können.

Die Anwendung der erfindungsgemäß verwendbaren Wirkstoffe geschieht auf diesem Gebiet in bekannter Weise wie durch orale Anwendung in Form von beispielsweise Tabletten, Kapseln, Tränken, Granulaten, durch dermale bzw. äußerliche Anwendung in Form beispielsweise des Tauchens (Dippen), Sprühens (Sprayen), Aufgießens (pour-on and spot-on) und des Einpuderns sowie durch parenterale Anwendung in Form beispielsweise der Injektion sowie ferner durch das "feed-through"-Verfahren. Daneben ist auch eine Anwendung als Formkörper (Halsband, Ohrmarke) möglich.

Die biologische Wirksamkeit der erfindungsgemäßen Verbindungen soll anhand der folgenden Beispiele erläutert werden.

 0233341

Herstellungsbeispiele:

Beispiel 1:

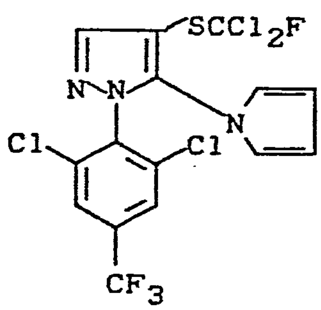

10 g (0.025 Mol) 5-Amino-4-dichlorfluormethylthio-1-(2,6-dichlor-4-trifluormethyl-phenyl)-pyrazol und 8,8 g (0.055 Mol) 2,5-Diethoxytetrahydrofuran werden in 60 ml Eisessig 4 Stunden unter Rückfluß erhitzt. Zur Aufarbeitung gießt man die erkaltete Reaktionsmischung in Wasser, extrahiert mehrfach mit Dichlormethan, trocknet über Natriumsulfat und entfernt das Lösungsmittel im Vakuum. Der Rückstand kristallisiert beim Verreiben mit Ligroin. Man erhält 8,5 g (71 % der Theorie) an 1-(2,4-Dichlor-4-trifluormethyl-phenyl)-5-(pyrrol-1-yl)-4-dichlorfluormethylsulfenyl-pyrazol vom Schmelzpunkt 93° C.

Herstellung der Ausgangsverbindungen

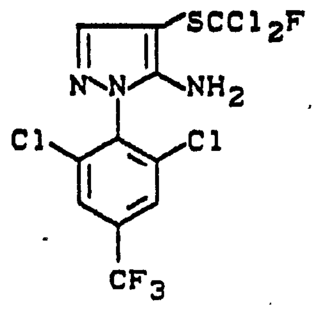

10 g (0.034 Mol) 5-Amino-1-(2,6-dichlor-4-trifluormethyl)-pyrazol werden in 50 ml Eisessig gelöst und bei Raumtemperatur tropfenweise mit 6,1 g (0.036 Mol) Dichlorfluormethansulfenylchlorid versetzt. Die Temperatur steigt bis auf ca. 40 °C an. Die Reaktionsmischung wird 2 Stunden gerührt und danach in eine Mischung aus 200 ml Wasser und 50 ml Dichlormethan eingetragen. Die organische Phase wird abgetrennt, die wässrige mit zweimal 20 ml Dichlormethan extrahiert. Die vereinigten organischen Phasen werden nacheinander mit Natriumhydrogencarbonat- und Kochsalzlösung gewaschen, über Magnesiumsulfat getrocknet und im Vakuum eingeengt.

Man erhält 13,6 g (94 % der Theorie) an 5-Amino-4-dichlorfluormethansulfenyl-1-(2,6-dichlor-4-trifluormethyl-phenyl)-pyrazol vom Schmelzpunkt 100 °C-103 °C.

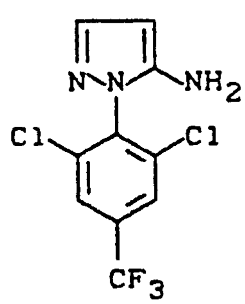

24,5 g (0.1 Mol) 2,6-Dichlor-4-trifluormethylphenylhydrazin und 20 mg Ethylendiamin-tetraessigsäure-Dinatriumsalz (=Titriplex III) in 150 ml Methanol werden bei Rückflußtemperatur tropfenweise mit 25 ml (27,6g/ 0.3 Mol) 2-Chlor-acrylnitril versetzt. Nach beendeter Zugabe erhitzt man weitere 8 Stunden auf Rückflußtemperatur, tropft dann 9 ml (0.16 Mol) 96 %ige Schwefelsäure zu und erhitzt weitere 6 Stunden auf Rückflußtemperatur. Die abgekühlte Reaktionsmischung wird mit 33,5 g (0.3 Mol) wasserfreiem Natriumcarbonat versetzt. Nach 4 Stunden entfernt man das Lösungsmittel im Vakuum, nimmt den Rückstand in 500 ml Wasser auf und rührt 10 Stunden bei Raumtemperatur. Man filtriert den ausgefallenen Niederschlag ab, wäscht mit Wasser nach und trocknet im Vakuum bei 50 °C.

Man erhält 28,5 g (96 % der Theorie) 5-Amino-1-(2,6-dichlor-4-trifluormethylphenyl)-pyrazol vom Schmelzpunkt 103 °C - 105 °C.

<u>Beispiel 2:</u>

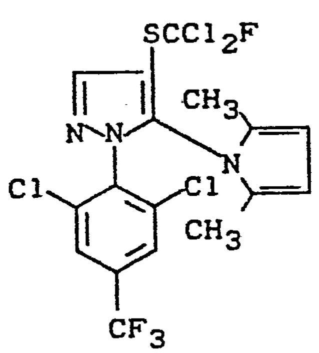

5 g (0,013 Mol) 5-Amino-4-dichlorfluormethylthio-1-(2,6-dichlor-4-trifluormethyl-phenyl)-pyrazol und 3 g (0,026 Mol) Acetonylaceton werden zusammen mit 0,5 g p-Toluolsulfonsäure in 150 ml Toluol 24 Stunden über einen Wasserabscheider unter Rückfluß erhitzt. Zur Aufarbeitung entfernt man das Lösungsmittel im Vakuum. Man erhält 6.0 g (94 % der Theorie) an 1-(2,6-Dichlor-4-trifluormethyl-phenyl)-5-(2,5-dimethyl-pyrrol-1-yl)-4-dichlorfluormethylsulfenyl-pyrazol vom Schmelzpunkt 72 °C - 75 °C.

Beispiel 3:

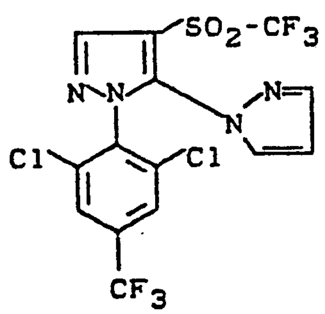

Zu 3 g (0.0068 Mol) 5-Hydrazino-4-(trifluormethylsulfonyl)-1-(2,6-dichlor-4-trifluormethyl-phenyl)-pyrazol in 7 ml Ethanol gibt man bei 20 °C bis 25 °C nacheinander 1,2 ml (0.0078 Mol) 1,1,3,3-Tetramethoxypropan und 0,2 ml (0.0034 Mol) 96-prozentige Schwefelsäure. Man erhitzt die Reaktionsmischung für 2 Stunden auf Rückflußtemperatur, kühlt ab, neutralisiert mit 0,36 g (0.0034 Mol) Natriumcarbonat und entfernt das Lösungsmittel im Vakuum. Der Rückstand wird in Wasser suspendiert, filtriert, nochmals mit Wasser gewaschen und getrocknet. Man erhält 2,5 g (77 % der Theorie) an 1-(2,6-Dichlor-4-trifluormethyl-phenyl)-5-(pyrazol-1-yl)

-4-trifluormethylsulfonyl-pyrazol vom Schmelzpunkt 127 °C bis 133 °C.

In entsprechender Weise und gemäß den allgemeinen Angaben zur Herstellung erhält man die folgenden 5-Heterocyclyl-1-aryl-pyrazole der allgemeinen Formel (I):

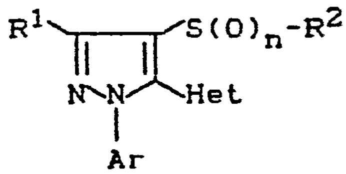

(I)

| Bsp. Nr. | $R^1$ | $-S(O)_n-R^2$ | Het | Ar | Schmelzpunkt/°C |
|---|---|---|---|---|---|
| 4 | H | $-SCCl_2F$ | 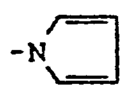 | 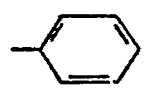 | 118-122 °C |
| 5 | H | $-SCF_3$ | 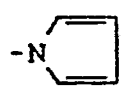 | 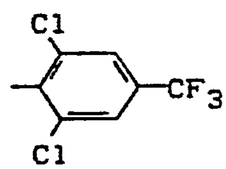 | 59-63 °C |
| 6 | H | $-SCCl_2F$ | 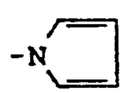 | 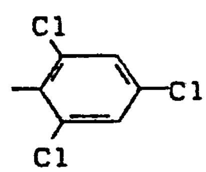 | 97-100 °C |
| 7 | H | $-SCCl_2F$ | 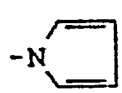 | 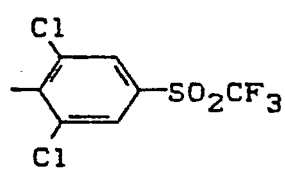 | 90-96 °C |

| Bsp. Nr. | $R^1$ | $-S(O)_n-R^2$ | Het | Ar | Schmelz-punkt/°C |
|---|---|---|---|---|---|
| 8 | H | $-SO_2-CCl_2F$ | 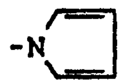 | 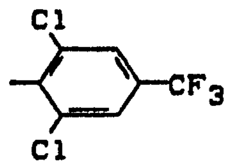 | 122 °C |
| 9 | H | $-SCF_3$ | 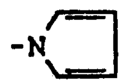 | 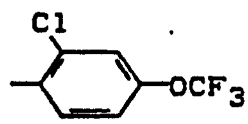 | Kp 150 °C/ 0.4 mbar |
| 10 | H | $-SCCl_2F$ | 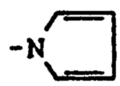 | 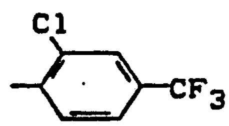 | $^1$H-NMR*): 6,24; 6,66 |
| 11 | H | $-SCF_3$ | 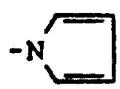 | 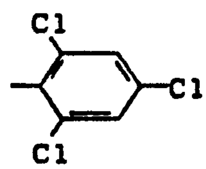 | $^1$H-NMR*): 6,19; 6,69 |
| 12 | H | $-SCCl_2F$ | 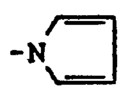 | 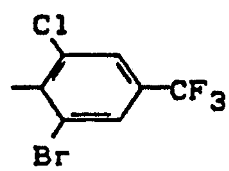 | 102-104 °C |
| 13 | H | $-SCCl_2F$ | 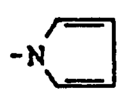 | 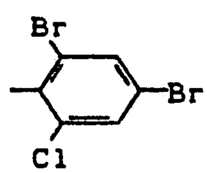 | 117-121 °C |

| Bsp. Nr. | $R^1$ | $-S(O)_n-R^2$ | Het | Ar | Schmelz-punkt/°C |
|---|---|---|---|---|---|
| 14 | H | $-SCCl_2F$ | 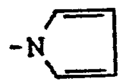 | 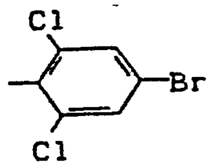 | 113-118 °C |
| 15 | H | $-S(O)-CCl_2F$ | 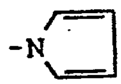 | 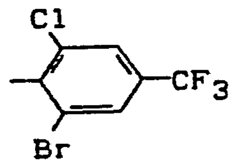 | 111-118 °C |
| 16 | H | $-SO_2-CCl_2F$ | 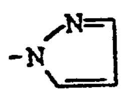 | 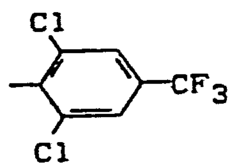 | 145-150 °C |
| 17 | H | $-S-CClF_2$ | 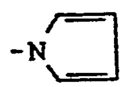 | 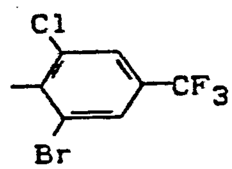 | 93-98 °C |
| 18 | H | $-S(O)-CF_3$ | 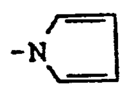 | 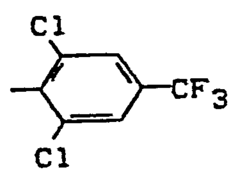 | 75-79 °C |
| 19 | H | $-SO_2-CF_3$ | 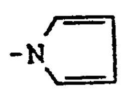 | 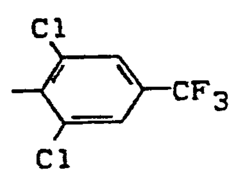 | 62-66 °C |

| Bsp. Nr. | $R^1$ | $-S(O)_n-R^2$ | Het | Ar | Schmelzpunkt/°C |
|---|---|---|---|---|---|
| 20 | $CH_3$ | $-SCCl_2F$ | 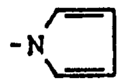 | 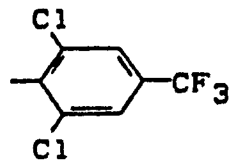 | $^{1}$H-NMR$^{*)}$: 6,23; 6,74 |
| 21 | $CH_3$ | $-SCF_3$ | 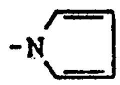 | 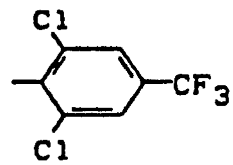 | $^{1}$H-NMR$^{*)}$: 6,22; 6,72 |
| 22 | H | $-SCH_3$ | 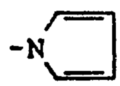 | 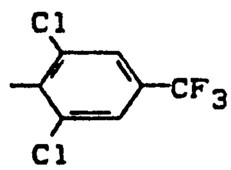 | 54-56 °C |
| 23 | H | $-SC_2H_5$ | 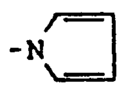 | 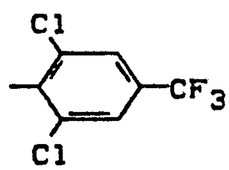 | 81 °C |
| 24 | H | $-SO_2-C_2H_5$ | 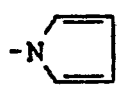 | 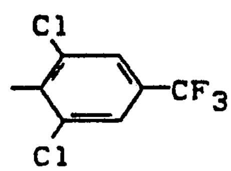 | 110 °C |
| 25 | $CH_3$ | $-S-C_2H_5$ | 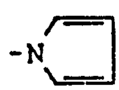 | 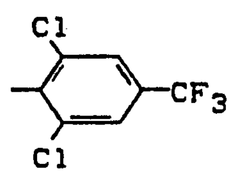 | 62 °C |

| Bsp. Nr. | $R^1$ | $-S(O)_n-R^2$ | Het | Ar | Schmelzpunkt/°C |
|---|---|---|---|---|---|
| 26 | $CH_3$ | $-S-CF_3$ | 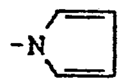 | 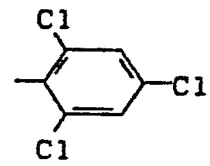 | $^1H$-NMR*): 6,24; 6,76 |
| 27 | $CH_3$ | $-S-CCl_2F$ | 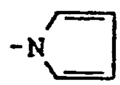 | 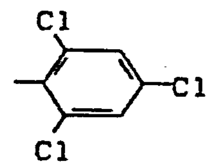 | $^1H$-NMR*): 6,16; 6,70 |
| 28 | $CH_3$ | $-S-CClF_2$ | 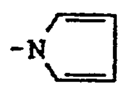 | 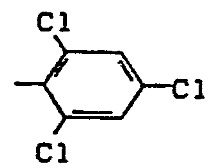 | $^1H$-NMR*): 6,17; 6,67 |
| 29 | $CH_3$ | $-SCF_3$ | 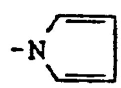 | 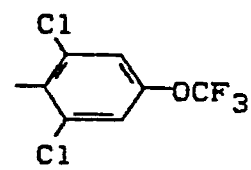 | $^1H$-NMR*): 6,17; 6,70 |
| 30 | $CH_3$ | $-S-CCl_2F$ | 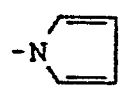 | 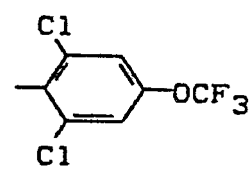 | $^1H$-NMR*): 6,18;6,71 |
| 31 | $CH_3$ | $-S-CClF_2$ | 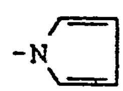 | 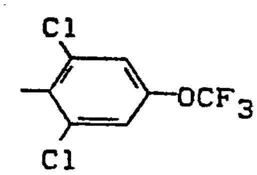 | $^1H$-NMR*): 6,18; 6,70 |

| Bsp. Nr. | $R^1$ | $-S(O)_n-R^2$ | Het | Ar | Schmelzpunkt/°C |
|---|---|---|---|---|---|
| 32 | H | $-S-CClF_2$ | 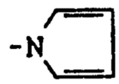 | 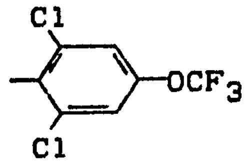 | Kp 200 °C/ 0.01 mbar |
| 33 | $CH_3$ | $-S-CClF_2$ | 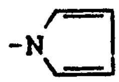 | 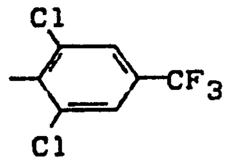 | $^1$H-NMR*): 6,17; 6,67 |
| 34 | H | $-SO_2-CClF_2$ | 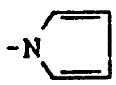 | 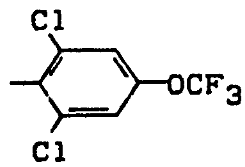 | Kp 165 °C/ 0.01 mbar |
| 35 | $CH_3$ | $-S-CCl_2F$ | 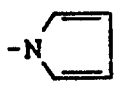 | 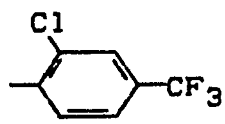 | Öl |
| 36 | $CH_3$ | $-S-CClF_2$ | 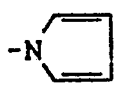 | 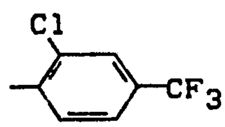 | Öl |
| 37 | $CH_3$ | $-S-CF_3$ | 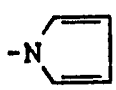 | 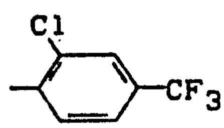 | Öl |
| 38 | $CH_3$ | $-S(O)-CF_3$ | 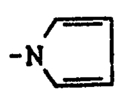 | 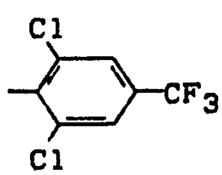 | Fp 96-97° C |

| Bsp. Nr. | $R^1$ | $-S(O)_n-R^2$ | Het | Ar | Schmelzpunkt/° C |
|---|---|---|---|---|---|
| 39 | $CH_3$ | $-SO_2-CF_3$ | 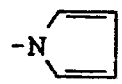 | 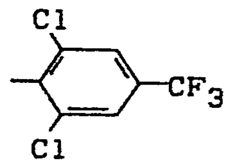 | Öl |
| 40 | H | $-S(O)-CCl_2F$ | 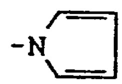 | 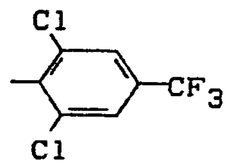 | Fp 123-124° C |
| 41 | H | $-S-CClF_2$ | 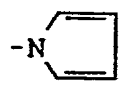 | 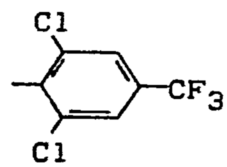 | Fp 97-98° C |
| 42 | H | $-S-CClF_2$ | 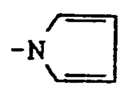 | 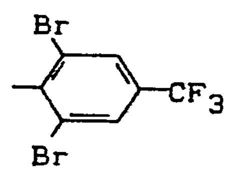 | Fp 106-109° C |
| 43 | H | $-S-CF_3$ | 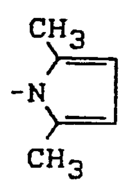 | 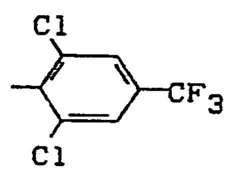 | Öl |

*) Die $^1$H-NMR-Spektren wurden in $CDCl_3$ aufgenommen mit Tetramethylsilan (TMS) als innerem Standard. Angegeben sind δ-Werte in ppm.

Herstellung der Ausgangsverbindungen der Formel IV

Beispiel IV-1:

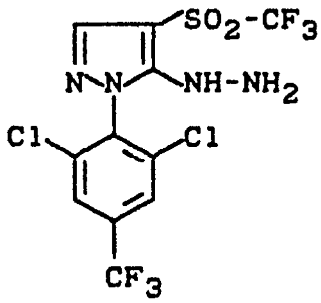

Zu 10 g (0.02 Mol) 5-Brom-1-(2,6-dichlor-4-trifluormethyl-phenyl)-4-trifluormethylsulfonyl-pyrazol in 140 ml Dioxan gibt man bei Raumtemperatur 2,5 ml (0.065 Mol) Hydrazinhydrat und erwärmt unter Rühren 4 Stunden lang auf 60 °C. Zur Aufarbeitung entfernt man das Lösungsmittel im Vakuum und reinigt das zurückbleibende Öl chromatographisch (Kieselgel, Laufmittel: Dichlormethan). Man erhält 6,5 g (72 % der Theorie) an 1-(2,6-Dichlor-4-trifluormethyl-phenyl)-5-hydrazino-4-trifluormethylsulfonyl-pyrazol vom Schmelzpunkt 154 °C.

Herstellung der Ausgangsverbindung:

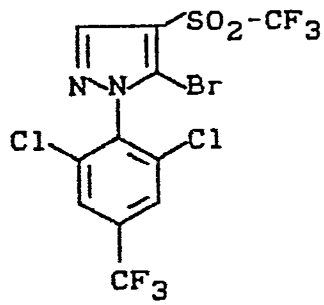

Eine Suspension von 28 g (0.065 Mol) 5-Amino-1-(2,6-dichlor-4-trifluormethyl-phenyl)-4-trifluormethylsulfonyl-pyrazol in 70 ml Bromoform gibt man bei 20 °C bis 25 °C zu einer Lösung von 15,5 ml (0.13 Mol) t-Butylnitrit in 30 ml Bromoform. Dabei steigt die Temperatur auf 40 °C an. Man rührt 15 Stunden bei Raumtemperatur nach, setzt 200 ml Dichlormethan zu, wäscht nacheinander mit wässriger Natriumhydrogencarbonat, Natriumthiosulfat und Natriumchloridlösung, trocknet die organische Phase über Magnesiumsulfat und entfernt das Lösungsmittel im Vakuum. Das zurückbleibende Öl wird chromatographisch (Kieselgel, Laufmittel: Dichlormethan) gereinigt. Man erhält 13,8 g (43 % der Theorie) an 5-Brom-1-(2,6-dichlor-4-trifluormethyl-phenyl)-4-trifluormethylsulfonyl-pyrazol vom Schmelzpunkt 90 °C bis 91 °C.

In entsprechender Weise und gemäß den allgemeinen Angaben zur Herstellung erhält man die folgenden 5-Hydrazino-1-aryl-pyrazole der allgemeinen Formel (IV):

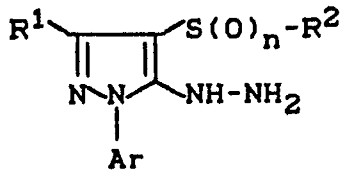

(IV)

| Bsp. Nr. | $R^1$ | $-S(O)_n-R^2$ | Ar | Schmelzpunkt/°C |
|---|---|---|---|---|
| IV-2 | H | $-SO_2-CCl_2F$ | 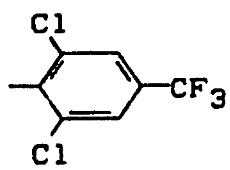 | 172-173 °C |
| IV-3 | H | $-SO_2-CCl_2F$ | 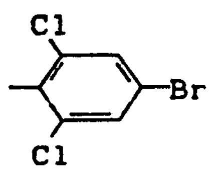 | 153-168 °C |
| IV-4 | H | $-SCCl_2F$ | 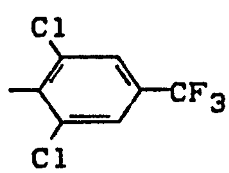 | |

Anwendungsbeispiele:

In den folgenden Anwendungsbeispielen wurden die nachstehend aufgeführten Verbindungen als Vergleichssubstanz eingesetzt:

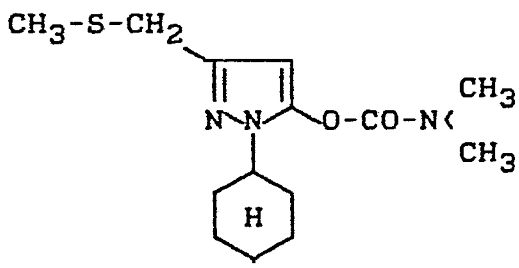

(A)

1-Cyclohexyl-5-[N,N-(dimethyl)-carbamoyloxy]-3-methylthiomethyl-pyrazol (bekannt aus DE-OS 2 839 270);

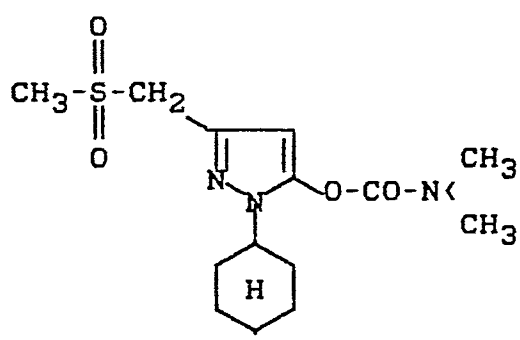

(B)

1-Cyclohexyl-5-[N,N-(dimethyl)-carbamoyloxy]-3-methylsulfonylmethyl-pyrazol (bekannt aus DE-OS 2 839 270).

**Beispiel A**

Phaedon-Larven-Test

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käferlarven abgetötet wurden; 0 % bedeutet, daß keine Käferlarven abgetötet wurden.

Bei diesem Test zeigen z.B. die folgenden Verbindungen der Herstellungsbeispiele überlegene Wirksamkeit gegenüber dem Stand der Technik: 1, 2, 5, 8, 10, 12, 15, 16, 17, 18, 19, 34.

Beispiel B

Plutella-Test

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigen z.B. die folgenden Verbindungen der Herstellungsbeispiele überlegene Wirksamkeit gegenüber dem Stand der Technik: 1, 2, 5, 8, 10, 12, 15, 16, 17, 18, 19, 34.

0233341

Beispiel C

Grenzkonzentrations-Test/Bodeninsekten

| | |
|---|---|
| Testinsekt: | Phorbia antiqua-Maden im Boden |
| Lösungsmittel: | 3 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 1, 5, 10, 12, 19, IV-2.

Beispiel D

Grenzkonzentrations-Test / Wurzelsystemische Wirkung

Testinsekt: Phaedon cochleariae-Larven
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle

Testtiere abgetötet sind und 0 %, wenn noch genau so viele Testinsekten leben wie bei der unbehandelten Kontrolle.

Bei diesem Test zeigt z.B. die folgende Verbindung der Herstellungsbeispiele überlegene Wirkung gegenüber dem Stand der Technik: 5.

Beispiel E

$LD_{100}$-Test

Testtiere: Musca domestica-Maden (resistent)
Zahl der Testtiere: 25
Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten geringeren Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

14 Tage nach dem Ansetzen der Versuche wird nach Abbott die Zahl der aus den Maden schlüpfenden Fliegen kontrolliert und die Verhinderung des Schlüpfens in % bestimmt.

Bei diesem Test zeigen z.B. die folgenden Verbindungen der Herstellungsbeispiele überlegene Wirkung gegenüber dem Stand der Technik: 1, 2, 3, 5, 8, 9, 10, 11, 12, 16, 17, 18, 19, 20, 21, 22 und 23 sowie IV-2, IV-4.

Beispiel F

$LD_{100}$-Test

Testtiere: Sitophilus granarius
Lösungsmittel: Aceton

2 Gewichtsteile Wirkstoff werden in 1000 Volumenteilen Lösungsmittel aufgenmommen. Die so erhaltene Lösung wird mit weiterem Lösungsmittel auf die gewünschten Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

Der Zustand der Testtiere wird 3 Tage nach Ansetzen der Versuche kontrolliert. Bestimmt wird die Abtötung in %. Dabei bedeutet 100 %, daß alle Testtiere abgetötet wurden: 0 % bedeutet, daß keine Testtiere abgetötet wurden.

Bei diesem Test zeigen z.B. die folgenden Verbindungen der Herstellungsbeispiele überlegene Wirkung gegenüber dem Stand der Technik: 1, 2, 3, 5, 9, 10, 11, 12, 14, 16, 17, 18, 19, 20, 21, 22, 23, 26, 27, 28, 29, 30, 31, 32, 33, 34, IV-1 und IV-2.

Beispiel G

Test mit Lucilia cuprina-Larven (OP-res. Goondiwindi-Stamm)

Emulgator: 35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Lösungsmittelgemisches und verdünnt das so erhaltene Konzentrat mit Wasser auf die jeweils gewünschte Konzentration.

Etwa 20 Lucilia cuprina res.-Larven werden in ein Teströhrchen gebracht, welches ca. 1 $cm^2$ Pferdefleisch und 0,5 ml der Wirkstoffzubereitung enthält. Nach 24 Stunden wird der Abtötungsgrad bestimmt.

Bei diesem Test zeigen z.B. die folgenden Verbindungen der Herstellungsbeispiele überlegene Wirkung gegenüber dem Stand der Technik: 1 und 2.

Patentansprüche

1. 5-Heterocyclyl-1-aryl-pyrazole der allgemeinen Formel (I)

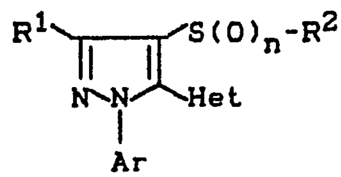

(I)

in welcher

$R^1$ für Wasserstoff, Alkyl oder Halogenalkyl steht,

$R^2$ für Alkyl, Alkenyl, Alkinyl, Cycloalkyl, Halogenalkyl, Halogenalkenyl, für gegebenenfalls substituiertes Aralkyl oder für gegebenenfalls substituiert Aryl steht,

Ar für jeweils gegebenenfalls substituiertes Phenyl oder Pyridyl steht,

Het für einen Heterocyclus der Formel

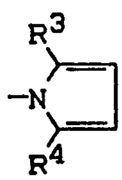 , 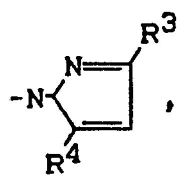 , oder 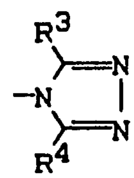 steht und

0233341

n für eine Zahl 0, 1 oder 2 steht,

wobei

$R^3$ und $R^4$ unabhängig voneinander jeweils für Wasserstoff, Alkyl oder Phenyl stehen.

2. 5-Heterocyclyl-1-aryl-pyrazole der allgemeinen Formel (I) gemäß Anspruch 1, in welcher

$R^1$ für jeweils geradkettiges oder verzweigtes Alkyl oder Halogenalkyl mit 1 bis 4 Kohlenstoffatomen und gegebenenfalls 1 bis 9 gleichen oder verschiedenen Halogenatomen oder für Wasserstoff steht,

$R^2$ für jeweils geradkettiges oder verzweigtes Alkyl, Alkenyl oder Alkinyl mit jeweils bis zu 8 Kohlenstoffatomen, für jeweils geradkettiges oder verzweigtes Halogenalkyl oder Halogenalkenyl mit jeweils bis zu 8 Kohlenstoffatomen und bis zu 17 gleichen oder verschiedenen Halogenatomen, für Cycloalkyl mit 3 bis 7 Kohlenstoffatomen oder für jeweils gegebenenfalls einfach oder mehrfach, gleich oder verschieden substituiertes Phenylalkyl oder Phenyl mit gegebenenfalls 1 bis 4 Kohlenstoffatomen im geradkettigen oder verzweigten Alkylteil steht, wobei als Phenylsubstituenten jeweils in Frage kommen: Halogen, Cyano, Nitro, jeweils geradkettiges oder verzweigtes Alkyl, Alkoxy, Alkylthio,

0233341

Alkylsulfinyl, Alkylsulfonyl, Halogenalkyl, Halogenalkoxy, Halogenalkylthio, Halogenalkylsulfinyl oder Halogenalkylsulfonyl mit jeweils 1 bis 4 Kohlenstoffatomen in den einzelnen Alkylteilen und gegebenenfalls 1 bis 9 gleichen oder verschiedenen Halogenatomen,

Ar für jeweils gegebenenfalls einfach oder mehrfach, gleich oder verschieden substituiertes Phenyl, 2-Pyridyl, 3-Pyridyl oder 4-Pyridyl steht, wobei als Substituenten in Frage kommen: Cyano, Nitro, Halogen, jeweils geradkettiges oder verzweigtes Alkyl, Alkoxy oder Alkoxycarbonyl mit jeweils bis zu 4 Kohlenstoffatomen, außerdem jeweils geradkettiges oder verzweigtes Halogenalkyl oder Halogenalkoxy mit jeweils bis zu 4 Kohlenstoffatomen und bis zu 9 gleichen oder verschiedenen Halogenatomen oder ein Rest $-S(O)_m-R^7$

wobei

$R^7$ für Amino, oder für jeweils geradkettiges oder verzweigtes Alkyl, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen steht und

m für eine Zahl 0, 1 oder 2 steht,

n ebenfalls für eine Zahl 0, 1 oder 2 steht und

Het für einen Rest

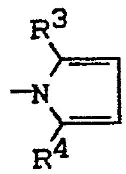 , 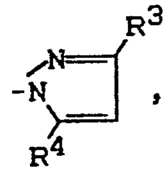 , oder 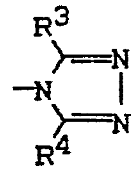

steht, wobei

$R^3$ und $R^4$ unabhängig voneinander jeweils für Wasserstoff, für geradkettiges oder verzweigtes Alkyl mit 1 bis 4 Kohlenstoffatomen oder für Phenyl stehen.

3. 5-Heterocyclyl-1-aryl-pyrazole der allgemeinen Formel (I) gemäß Anspruch 1 bis 2, dadurch gekennzeichnet, daß

$R^1$ für Wasserstoff, Methyl, Ethyl, n- oder i-Propyl oder Trifluormethyl steht,

$R^2$ für Methyl, Ethyl, n- oder i-Propyl, n-, i-, s- oder t-Butyl, n- oder i-Pentyl, n- oder i-Hexyl, Allyl, Propenyl, Butenyl, Propargyl, Butinyl, Cyclopropyl, Cyclopentyl, Cyclohexyl, Chlormethyl, Difluormethyl, Difluorchlormethyl, Fluordichlormethyl, Trifluormethyl, Pentafluorethyl, Pentachlorethyl, Fluortetrachlorethyl, Difluortrichlorethyl, Trifluordichlorethyl, Tetrafluorchlorethyl, Heptafluorpropyl, Chlorethyl, Brommethyl, Chlorpropyl, Chlorpropenyl, Dichlorpropenyl, Chlorbutenyl oder für jeweils gegebenenfalls ein- bis dreifach, gleich oder verschieden substituiertes Phenyl, Benzyl oder Phenylethyl steht, wobei als Phenylsubstituenten in Frage kommen:

Fluor, Chlor, Brom, Iod, Cyano, Nitro, Methyl, Ethyl, Methoxy, Methylthio, Trifluormethyl, Methylsulfinyl, Methylsulfonyl, Trifluormethoxy, Trifluormethylthio, Trifluormethylsulfinyl oder Trifluormethylsulfonyl,

Ar für jeweils gegebenenfalls ein- bis fünffach, gleich oder verschieden substituiertes Phenyl, 2-Pyridyl, 3-Pyridyl oder 4-Pyridyl steht, wobei als Substituenten in Frage kommen:
Cyano, Nitro, Fluor, Chlor, Brom, Iod, Methyl, Ethyl, n- und i-Propyl, n-, i-, s- und t-Butyl, Methoxy, Ethoxy, Methoxycarbonyl, Ethoxycarbonyl, Trifluormethyl, Trichlormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_m-R^7$ wobei

$R^7$ für Amino, Methylamino, Ethylamino, Dimethylamino, Diethylamino, Fluordichlormethyl, Difluormethyl, Tetrafluorethyl, Trifluorchlorethyl, Trifluormethyl, Methyl oder Ethyl steht,

m für eine Zahl 0, 1 oder 2 steht,

n ebenfalls für eine Zahl 0, 1 oder 2 steht und

Het für einen Heterocyclus der Formel

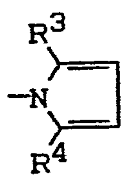 , 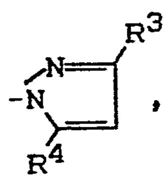 , oder 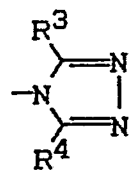 steht,

wobei

$R^3$ und $R^4$ unabhängig voneinander jeweils für Wasserstoff, Methyl, Ethyl, n- oder i-Propyl oder Phenyl stehen.

4. Verfahren zur Herstellung von 5-Heterocyclyl-1-arylpyrazolen der allgemeinen Formel (I)

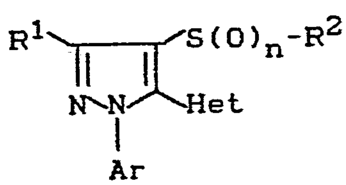 (I)

in welcher

$R^1$ für Wasserstoff, Alkyl oder Halogenalkyl steht,

$R^2$ für Alkyl, Alkenyl, Alkinyl, Cycloalkyl, Halogenalkyl, Halogenalkenyl, für gegebenenfalls substituiertes Aralkyl oder für gegebenenfalls substituiertes Aryl steht,

Ar für jeweils gegebenenfalls substituiertes Phenyl oder Pyridyl steht,

Het für einen Heterocyclus der Formel

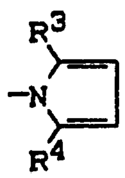 , 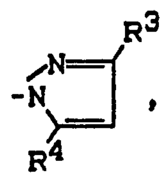 , oder 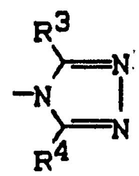 steht und

n für eine Zahl 0, 1 oder 2 steht,

wobei

$R^3$ und $R^4$ unabhängig voneinander jeweils für Wasserstoff, Alkyl oder Phenyl stehen,

dadurch gekennzeichnet,

a) daß man zum Erhalt von 5-Pyrrolyl-1-arylpyrazolen der Formel (Ia)

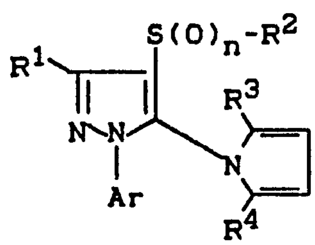

(Ia)

in welcher

$R^1$, $R^2$, $R^3$, $R^4$, n und Ar die oben angegebene Bedeutung haben,

5-Amino-1-aryl-pyrazole der Formel (II)

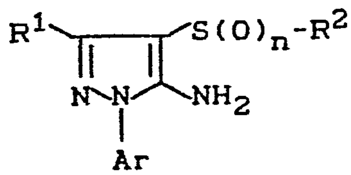

(II)

in welcher

$R^1$, $R^2$, Ar und n die oben angegebene Bedeutung haben,

α) mit 1,4-Dicarbonylverbindungen der Formel (IIIa)

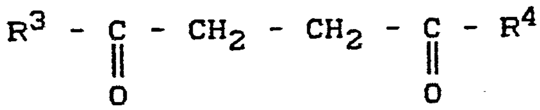

(IIIa)

in welcher

$R^3$ und $R^4$ die oben angegebene Bedeutung haben,

oder

β) mit deren Acetalen oder Ketalen der Formel (IIIb),

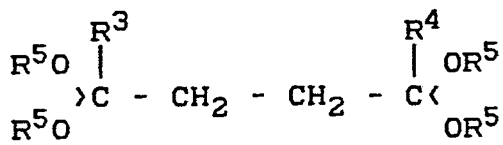

(IIIb)

in welcher

$R^3$ und $R^4$ die oben angegebene Bedeutung haben und

$R^5$ für Alkyl steht,

oder

γ) mit deren cyclischen Acetalstrukturen der Formel (IIIc)

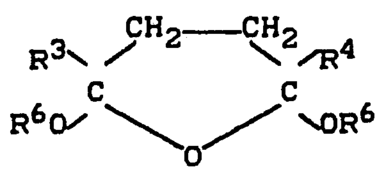

(IIIc)

in welcher

$R^3$ und $R^4$ die oben angegebene Bedeutung haben und

$R^6$ für Alkyl steht,

jeweils gegebenenfalls in Gegenwart eines Verdünnungsmittels und gegebenenfalls in Gegenwarteines Reaktionshilfsmittels umsetzt;

b) oder daß man zum Erhalt von 5-(Pyrazol-1-yl)-1-arylpyrazolen der Formel (Ib),

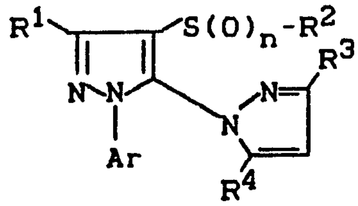

(Ib)

in welcher

$R^1$, $R^2$, $R^3$, $R^4$, Ar und n die oben angegebene Bedeutung haben,

5-Hydrazino-1-aryl-pyrazole der Formel (IV)

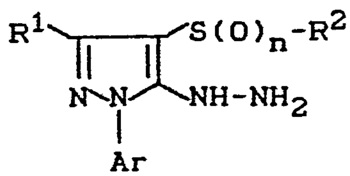

(IV)

in welcher

$R^1$, $R^2$, Ar und n die oben angegebene Bedeutung haben,

α) mit 1,3-Dicarbonylverbindungen der Formel (Va),

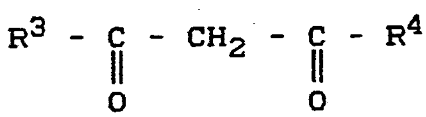

(Va)

in welcher

$R^3$ und $R^4$ die oben angegebene Bedeutung haben,

oder

β) mit deren Acetalen oder Ketalen der Formel (Vb),

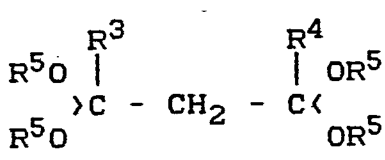

(Vb)

in welcher

$R^3$, $R^4$ und $R^5$ die oben angegebene Bedeutung haben,

jeweils gegebenenfalls in Gegenwart eines Verdünnungsmittels und gegebenenfalls in Gegenwart eines Reaktionshilfsmittels umsetzt;

c) oder daß man zum Erhalt von 5-(1,3,4-Triazol-1-yl)-1-aryl-pyrazolen der Formel (Ic),

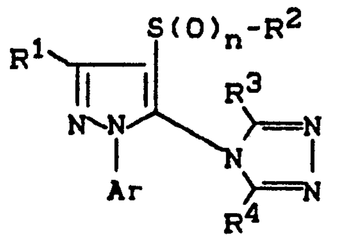

(Ic)

in welcher

$R^1$, $R^2$, $R^3$, $R^4$, Ar und n die oben angegebene Bedeutung haben,
5-Amino-1-aryl-pyrazole der Formel (II),

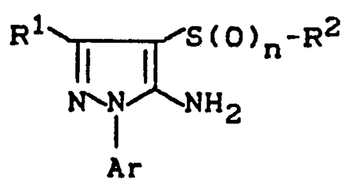

(II)

in welcher

$R^1$, $R^2$, Ar und n die oben angegebene Bedeutung haben,

mit Diacyl-Hydrazinen der Formel (VI),

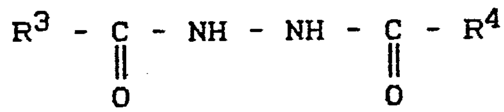 (VI))

in welcher

$R^3$ und $R^4$ die oben angegebene Bedeutung haben,

gegebenenfalls in Gegenwart eines Verdünnungsmittels umsetzt.

5) Schädlingsbekämpfungsmittel, gekennzeichnet durch einen Gehalt an mindestens einem 5-Heterocyclyl-1-aryl-pyrazol der Formel (I).

6) Insektizides Mittel, gekennzeichnet durch einen Gehalt an mindestens einem 5-Heterocyclyl-1-aryl-pyrazol der Formel (I).

7) Verfahren zur Bekämpfung von tierischen Schädlingen, dadurch gekennzeichnet, daß man 5-Heterocyclyl-1-aryl-pyrazole der Formel (I) auf tierische Schädlinge und/oder ihren Lebensraum einwirken läßt.

8) Verwendung von 5-Heterocyclyl-1-aryl-pyrazolen der Formel (I) zur Bekämpfung von tierischen Schädlingen.

9) Verfahren zur Herstellung von Schädlingsbekämpfungsmitteln, dadurch gekennzeichnet, daß man 5-Heterocyclyl-1-aryl-pyrazole der Formel (I) mit Streckmitteln und/oder oberflächenaktiven Mitteln vermischt.

10) 5-Hydrazino-1-aryl-pyrazole der Formel (IV)

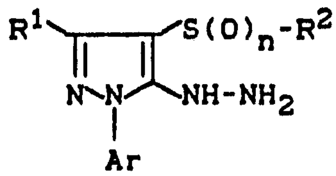

(IV)

in welcher

$R^1$ für Wasserstoff, Alkyl oder Halogenalkyl steht,

$R^2$ für Alkyl, Alkenyl, Alkinyl, Cycloalkyl, Halogenalkyl, Halogenalkenyl, für gegebenenfalls substituiertes Aralkyl oder für gegebenenfalls substituiertes Aryl steht,

Ar für jeweils gegebenenfalls substituiertes Phenyl oder Pyridyl steht, und

n für eine Zahl 0, 1 oder 2 steht.

11) Verfahren zur Herstellung von 5-Hydrazino-1-aryl-pyrazolen der Formel (IV)

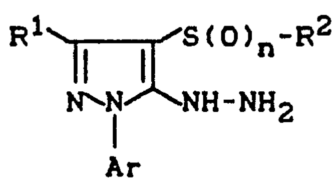

(IV)

in welcher

$R^1$ für Wasserstoff, Alkyl oder Halogenalkyl steht,

$R^2$ für Alkyl, Alkenyl, Alkinyl, Cycloalkyl, Halogenalkyl, Halogenalkenyl, für gegebenenfalls substituiertes Aralkyl oder für gegebenenfalls substituiertes Aryl steht,

Ar für jeweils gegebenenfalls substituiertes Phenyl oder Pyridyl steht, und

n für eine Zahl 0, 1 oder 2 steht,

dadurch gekennzeichnet, daß man 5-Amino-1-aryl-pyrazole der Formel (II)

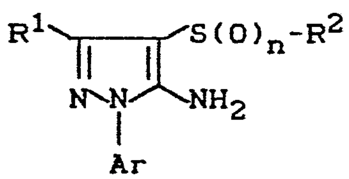

(II)

in welcher

$R^1$, $R^2$, Ar und n die oben angegebene Bedeutung haben,

zunächst in einer ersten Stufe mit Nitrit-Verbindungen der Formel (XIII),

$$R^9 - O - N = O \qquad (XIII)$$

in welcher

$R^9$ für Wasserstoff, Alkyl oder für ein Alkalimetallkation steht,

in Gegenwart einer Halogenwasserstoffsäure oder in Gegenwart eines Haloforms diazotiert und die so erhaltenen 5-Halogen-1-aryl-pyrazole der Formel (XIV)

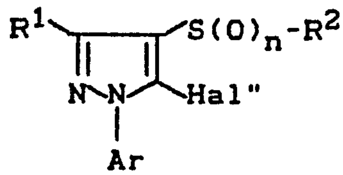

(XIV)

in welcher

$R^1$, $R^2$, Ar und n die oben angegebene Bedeutung haben und

Hal" für Halogen steht,

mit Hydrazinhydrat, gegebenenfalls in Gegenwart eines Verdünnungsmittels, umsetzt.

12. Schädlingsbekämpfungsmittel, gekennzeichnet durch einen Gehalt an mindestens einem 5-Hydrazino-1-aryl-pyrazol der Formel (IV) gemäß Anspruch 10.

13. Verfahren zur Herstellung von Schädlingsbekämpfungsmitteln, dadurch gekennzeichnet, daß man 5-Hydrazino-1-aryl-pyrazole der Formel (IV) mit Streckmitteln und/oder oberflächenaktiven Mitteln vermischt.

Europäisches Patentamt

# EUROPÄISCHER RECHERCHENBERICHT

0233341

Nummer der Anmeldung

EP 86 11 7017

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl 4) |
|---|---|---|---|
| A | US-A-3 534 058 (SANTILLI-OSDENE)<br>--- | | C 07 D 403/04<br>C 07 D 231/44<br>A 01 N 43/56<br>A 01 N 43/36<br>A 01 N 43/647 |
| P,A | EP-A-0 167 028 (BAYER)<br>--- | | |
| P,A | EP-A-0 201 852 (BAYER)<br>----- | | |
| | | | **RECHERCHIERTE SACHGEBIETE (Int. Cl.4)** |
| | | | C 07 D 403/00<br>C 07 D 231/00<br>A 01 N 43/00 |

Der vorliegende Recherchenbericht wurde für alle Patentansprüche erstellt.

| Recherchenort | Abschlußdatum der Recherche | Prüfer |
|---|---|---|
| DEN HAAG | 27-03-1987 | DE BUYSER I.A.F. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82